# EUROPÄISCHE PATENTANMELDUNG

(11) **EP 1 927 330 A1**
(43) Veröffentlichungstag der Anmeldung: **04.06.2008**
(21) Anmeldenummer: 07019787.6
(22) Anmeldetag: 10.10.2007
(51) Int. Cl.: A61F 11/12, A61F 9/02, G02C 5/22

(54) **Schutzeinrichtung, wie Arbeitsschutz-Brille, Bügelgehörschutz oder dergleichen**

(30) Priorität: 29.11.2006 DE 102006056219
(71) Anmelder: Uvex Arbeitsschutz GmbH, 90766 Fürth (DE)
(72) Erfinder: Wozniak, Cordula, 90429 Nürnberg (DE)
(74) Vertreter: Schneck, Herbert

(57) **Zusammenfassung**

Bei einer Schutzeinrichtung für den menschlichen Körper, insbesondere Bügelgehörschutz, Arbeitsschutz-Brille oder dergleichen, umfassend ein Gelenk zwischen zwei gelenkig zu verbindenden Teilen (12,13), welches durch ein Filmscharnier (14) in Form eines einstückig mit den zu verbindenden Teilen gespritzten Scharnierbandes gebildet ist, ist vorgesehen dass, zusätzlich zu dem ersten Scharnierband ein ebenfalls mit den zu verbindenden Teilen einstückig gespritztes zweites, einfaltbares Scharnierband (15) vorgesehen ist, welches im Gelenkbereich im Abstand zum ersten Scharnierband angeordnet ist.

## Beschreibung

Die Erfindung richtet sich auf eine Schutzeinrichtung für den menschlichen Körper, insbesondere einen Bügelgehörschutz, eine Arbeitsschutz-Brille oder dergleichen, umfassend ein Gelenk zwischen zwei gelenkig verbindenden Teilen, welches durch ein Filmscharnier in Form eines einstückig mit den zu verbindenden Teilen gespritzten Scharnierbandes gebildet ist.

Eine solche Schutzeinrichtung in Form eines Bügelgehörschutzes ist beispielsweise bekannt aus DE 296 11 562 U1.

Derartige bekannte Lösungen weisen den Nachteil auf, dass das Filmscharnier eine relativ geringe Torsionsfestigkeit besitzt, die die gesamte Anordnung instabil macht und ggf. auch zu einer nicht hinreichenden Haltbarkeit führt.

Hiervon ausgehend liegt der Erfindung die Aufgabe zugrunde, eine Schutzeinrichtung bzw. ein Filmscharnier zu schaffen, wobei im Gelenkbereich bei einfacher Herstellbarkeit eine hohe Torsionsstabilität erzielt wird.

Diese Aufgabe wird erfindungsgemäß dadurch gelöst, dass zusätzlich zu dem ersten Scharnierband ein ebenfalls mit den zu verbindenden Teilen einstückig gespritztes, zweites einfaltbares Scharnierband vorgesehen ist, welches im Gelenkbereich im Abstand zum ersten Scharnierband angeordnet ist.

Dieses zweite Scharnierband bildet zusammen mit dem ersten Scharnierband eine Art Kastenprofil mit hoher Drehstabilität. Während das erste Scharnierband in herkömmlicher Weise die zu verbindenden Teile unmittelbar aneinander anschließend verbindet, ist erfindungsgemäß das zweite Scharnierband mit einer Faltung versehen, welche beim Schließen des Gelenks, also in der gestreckten Position der zu verbindenden Teile, entweder eingefaltet nach außen vorsteht, oder sich eingefaltet in das Innere des Scharnierbereichs erstreckt.

Mit anderen Worten erstreckt sich das zweite Scharnierband gefaltet über den Gelenk-Öffnungsbereich, der durch das erste Scharnierband definiert wird.

Das zweite Scharnierband kann sich von den Außenkanten der zu verbindenden Teile im Scharnierbereich von dem ersten Scharnierband wegerstrecken, was allerdings den Nachteil mit sich bringt, dass im gestreckten Zustand das zweite Scharnierband nach außen vorsteht.

Deshalb ist vorzugsweise vorgesehen, dass die zu verbindenden Teile an ihren Stirnflächen im Gelenkbereich jeweils eine sich nach außen hin öffnende Schrägfläche aufweisen, wobei das zweite Scharnierband sich von den Übergangskanten zwischen Stirnseite und jeweiliger Schrägfläche nach innen wegerstreckt und im geöffneten Zustand des Gelenks eingefaltet im Inneren des Gelenks zuliegen kommt.

Um das Gelenk in einfacher und kostengünstiger Weise durch ein sogenanntes Auf-Zu-Spritzgießwerkzeug herstellen zu können, ist vorgesehen, dass das Gelenk keine sich in Richtung der Schwenkachsen vorspringenden Abschnitte aufweist.

Zur Erzielung einer Endlagenstabilität im gestreckten Zustand kann vorgesehen sein, dass an den Stirnseiten der gelenkig zu verbindenden Teile wenigstens ein Vorsprung, z. B. in Form eines Raststeges, und an dem jeweils anderen Teil wenigstens eine Rastausnehmung, z. B. in Form einer Rastnut, vorgesehen ist.

Die Erfindung richtet sich auch auf ein Filmscharnier, insbesondere für ein Körperschutzprodukt, wie Bügelgehörschutz oder Arbeitsschutz-Brille, umfassend wenigstens ein einstückig mit zwei gelenkig zu verbindenden Teilen gespritztes Gelenkband, welches sich dadurch auszeichnet, dass zusätzlich zu dem ersten Scharnierband ein ebenfalls mit den zu verbindenden Teilen einstückig gespritztes zweites, einfaltbares Scharnierband vorgesehen ist, welches im Gelenkbereich im Abstand zum ersten Scharnierband angeordnet ist.

Nachfolgend wird die Erfindung anhand eines bevorzugten Ausführungsbeispiels in Verbindung mit der Zeichnung näher erläutert. Dabei zeigen:
- Fig. 1: einen Bügelgehörschutz mit erfindungsgemäßen Filmscharnieren im geöffneten Gebrauchszustand,
- Fig. 2: den Bügelgehörschutz nach Fig. 1 im eingefalteten Zustand,
- Fig. 3: eine Arbeitsschutz-Brille mit erfindungsgemäßen Filmscharnieren und
- Fig. 4: eine perspektivische Darstellung eines erfindungsgemäßen Filmscharniers.

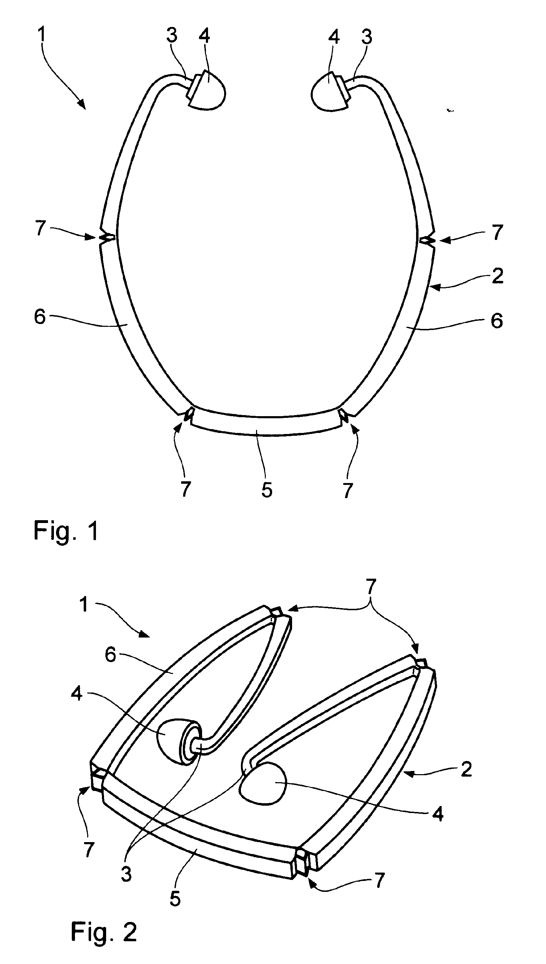

In Fig. 1 ist ein Bügelgehörschutz 1 schematisch dargestellt umfassend einen im Wesentlichen U-förmigen Bügel 2, an dessen freien abgekröpften Enden 3 Gehörschutzstöpsel 4 angeordnet sind.

Zwischen einem U-Boden 5 des U-förmigen Bügels 2 und den U-Schenkeln 6 ist jeweils ein Filmscharnier 7 ausgebildet, wobei ebenso jeder U-Schenkel durch ein Filmscharnier 7 geteilt ist, sodass der Gehörschutz 1, wie in Fig. 2 dargestellt, zur Aufbewahrung, z. B. einer Brusttasche, zusammengefaltet werden kann.

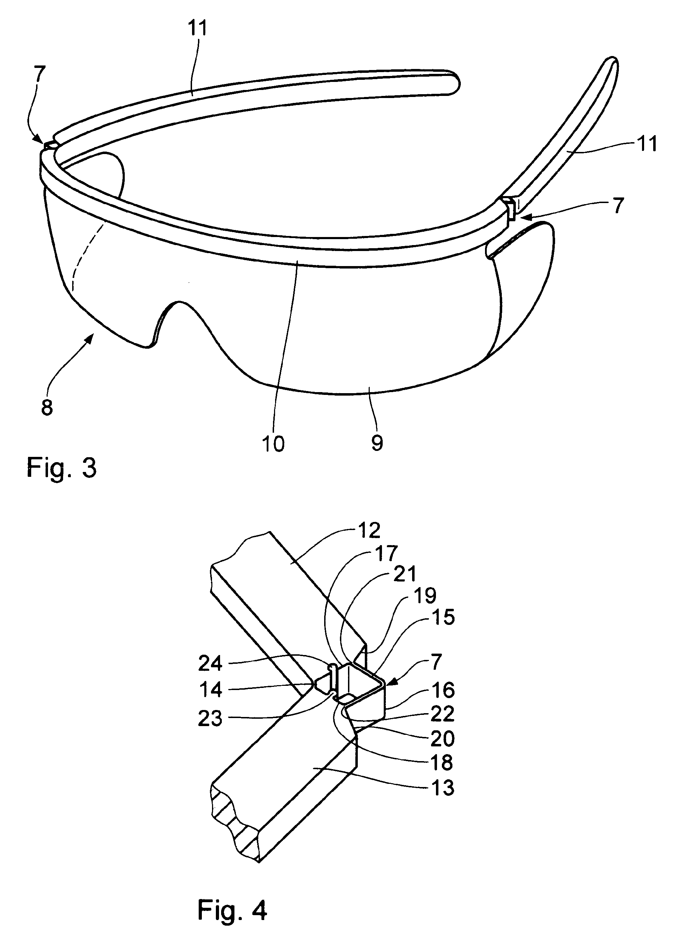

In Fig. 3 ist eine Arbeitsschutz-Brille 8 mit einer einstückigen durchgehenden Scheibe 9 und einem oberen Rahmenteil 10 schematisch dargestellt, wobei an beiden Enden des oberen Rahmenteils 10 Bügel 11 über ein Scharnierband 7 angelenkt sind.

In Fig. 4 ist das Filmscharnier 7 zwischen zwei zu verbindenden Teilen 12, 13 dargestellt. Diese zu verbindenden Teile 12, 13 können gebildet sein durch den U-Boden 5 und einen U-Schenkel 6 bei dem in Fig. 1 dargestellten Bügelgehörschutz oder durch das obere Rahmenteil 10 und einen Bügel 11 bei der Arbeitsschutz-Brille 8 gemäß Fig. 3. Das erfindungsgemäße Scharnier eignet sich durch seine geringe Bauhöhe und seine Beständigkeit gegen Torsion und der Möglichkeit einstückiger und kostengünstiger Fertigung auch für zahlreiche andere Anwendungen mit vergleichbaren Anforderungen.

Das in Fig. 4 dargestellte Filmscharnier 7 umfasst ein erstes mit den Teilen 12, 13 einstückig gespritztes Scharnierband 14, welches die Teile 12, 13 unmittelbar miteinander verbindet, und im Abstand hiervon ein zweites Scharnierband 15, welches eine sich im Ausführungsbeispiel nach außen erstreckende Faltung 16 aufweist. Das zweite Scharnierband 15 überbrückt auf diese Weise mit einer Falte den durch das erste Scharnierband 14 aufgespannten Gelenkbereich.

Die Stirnflächen 17, 18 der zu verbindenden Teile 12, 13 weisen Schrägflächen 19, 20 auf, welche mit den Stirnflächen 17, 18 jeweils eine Kante 21, 22 ausbilden. Das zweite Scharnierband 15 erstreckt sich von der ersten Kante 21 zur zweiten Kante 22 und kommt dementsprechend im gestreckten Zustand bündig nach außen abschließend zwischen den Schrägflächen 19, 20 zuliegen.

Zur Stabilisierung bzw. Arretierung des Filmscharniers 7 im gestreckten Gebrauchszustand ist ein Rastvorsprung 23 an dem ersten Teil 13 und eine Rastausnehmung 24 in Form einer Rastnut an dem zweiten Teil 12 vorgesehen, die formschlüssig und kraftschlüssig rastend ineinander greifen, wenn das Gelenk geschlossen wird bzw. in den gestreckten Zustand gebracht wird.

## Patentansprüche

1. Schutzeinrichtung für den menschlichen Körper, insbesondere Bügelgehörschutz, Arbeitsschutz-Brille oder dergleichen, umfassend ein Gelenk zwischen zwei gelenkig zu verbindenden Teilen, welches durch ein Filmscharnier in Form eines einstückig mit den zu verbindenden Teilen gespritzten Scharnierbandes gebildet ist, **dadurch gekennzeichnet, dass** zusätzlich zu dem ersten Scharnierband ein ebenfalls mit den zu verbindenden Teilen einstückig gespritztes zweites, einfaltbares Scharnierband vorgesehen ist, welches im Gelenkbereich im Abstand zum ersten Scharnierband angeordnet ist.

2. Schutzeinrichtung nach Anspruch 1, **dadurch gekennzeichnet, dass** das zweite Scharnierband sich über den Gelenk-Öffnungsbereich, der durch das erste Scharnierband definiert wird, erstreckt.

3. Schutzeinrichtung nach Anspruch 1, **dadurch gekennzeichnet, dass** das zweite Scharnierband sich von den Außenkanten der zu verbindenden Teile im Scharnierbereich von dem ersten Scharnierband wegerstreckt.

4. Schutzeinrichtung nach Anspruch 1, **dadurch gekennzeichnet, dass** die zu verbindenden Teile an ihren Stirnflächen im Gelenkbereich jeweils eine sich nach außen hin öffnende Schrägfläche aufweisen, wobei das zweite Scharnierband sich von den Übergangskanten zwischen Stirnseite und jeweiliger Fase nach innen wegerstreckt und im geöffneten Zustand des Gelenks eingefaltet zwischen den Fasen zu liegen kommt.

5. Schutzeinrichtung nach Anspruch 1, **dadurch gekennzeichnet, dass** das Gelenk keine sich in Richtung der Scharnierachsen vorspringenden Abschnitte aufweist.

6. Schutzeinrichtung nach Anspruch 1, **dadurch gekennzeichnet, dass** an den Stirnseiten der gelenkig zu verbindenden Teile wenigstens ein Vorsprung und an dem jeweils anderen Teil wenigstens eine Rastausnehmung zur Arretierung des Gelenks im offenen Zustand vorgesehen sind.

7. Filmscharnier, insbesondere für ein Körperschutzprodukt, wie Bügelgehörschutz oder Arbeitsschutz-Brille, umfassend wenigstens ein einstückig mit zwei gelenkig zu verbindenden Teilen gespritztes Gelenkband, **dadurch gekennzeichnet, dass** zusätzlich zu dem ersten Scharnierband ein ebenfalls mit den zu verbindenden Teilen einstückig gespritztes, zweites, einfaltbares Scharnierband vorgesehen ist, welches im Gelenkbereich im Abstand zum ersten Scharnierband angeordnet ist.
